# EUROPÄISCHE PATENTSCHRIFT

(11) **EP 2 676 641 B1**
(45) Veröffentlichungstag und Bekanntmachung des Hinweises auf die Patenterteilung: **16.12.2020**
(21) Anmeldenummer: 13159311.3
(22) Anmeldetag: 15.03.2013
(51) Int. Cl.: A61F 2/966, A61M 25/00, A61M 25/06, A61M 25/10

(54) **Einführvorrichtung**
Insertion device
Dispositif d'insertion

(30) Priorität: 18.06.2012 US 201261660831 P; 10.12.2012 US 201213709805
(43) Veröffentlichungstag der Anmeldung: 25.12.2013
(73) Patentinhaber: Biotronik AG, 8180 Bülach (CH)
(72) Erfinder: Schwitzer, Alwin, 8180 Bülach (CH); Fargahi, Amir, 8180 Bülach (CH)
(74) Vertreter: Galander, Marcus

(56) Entgegenhaltungen:
- EP-A1- 0 839 549
- US-A1- 2004 087 968
- US-A1- 2006 287 669
- US-A1- 2010 198 160

## Beschreibung

Die Erfindung betrifft eine Einführvorrichtung nach dem unabhängigen Patentanspruch 1. Weitere Ausführungsformen sind den davon abhängigen Ansprüchen 2 bis 5 zu entnehmen.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina-Implantate, zahnmedizinische Implantate, Implantate zum Gelenkersatz, Gefäßprotesen oder Stents.

Implantate werden vor einem Einführen in den Körper mit Kathetern verbunden, mit dessen Hilfe sie am Einsatzort genau platziert und definiert freigegeben werden können. Zum Einschleusen in den tierischen und/oder menschlichen Körper wird hierzu ein schlauchartiges Einführelement eingesetzt, durch welches das Implantat mittels der Einführvorrichtung geschoben wird. Um die Belastung der Gefäße beim Einschleusen des Implantats zu vermindern, ist aus der US 2010/0094392 A1 ein Einführelement bekannt, welches erst beim Passieren des Implantats auf den notwendigen größeren Durchmesser aufgeweitet wird. Das Einführelement besteht aus zwei bis drei koaxial angeordneten Lagen, wobei die äußere mit longitudinalen Schlitzen versehen ist, was die Vergrößerung des Durchmessers ermöglicht.

US 2004/0087968 A1 offenbart einen Einführschaft aus zwei oder mehr Lagen, wobei eine innere Lage aus einem Material mit einer höheren Shore-Härte und eine äußere Lage aus einem Material mit geringerer Shore-Härte ausgewählt ist.

EP 0 839 549 A1 offenbart einen Katheter zur Einführung in das menschliche Gefäßsystem. Der Katheter kann irreversibel von einem ersten Durchmesser auf einen zweiten Durchmesser expandiert werden.

US 2010/0198160 A1 offenbart ebenfalls einen Einführschaft.

US 2006/0287669 A1 offenbart einen Katheter mit einer Katheterspitze, die zum Wiedereinfangen eines Embolieschutzfilters geeignet ist.

Der Erfindung liegt die Aufgabe zugrunde, ein Einführelement als Teil einer Einführvorrichtung zum Einführen eines medizinischen Implantats anzugeben, das in seiner Herstellung vereinfacht ist und das eine verbesserte Flexibilität des Einführelements ermöglicht.

Eine weitere Aufgabe ist in der Bereitstellung einer entsprechenden Einführvorrichtung zu sehen.

Die Aufgabe wird erfindungsgemäß durch die Merkmale des unabhängigen Anspruchs 1 gelöst. Günstige Ausgestaltungen und Vorteile der Erfindung ergeben sich aus den weiteren Ansprüchen und der Beschreibung.

Es wird ein Einführelement als Teil einer Einführvorrichtung zum Einführen eines medizinischen Implantats vorgeschlagen, welches zum Zusammenwirken mit einer Einführvorrichtung zum Einführen eines medizinischen Implantats in einen menschlichen oder tierischen Körper ausgebildet ist. Das Einführelement besitzt eine Hülle, welche in Umfangsrichtung wenigstens zwei Arten von Umfangssegmenten mit unterschiedlicher Elastizität aufweist.

Vorteilhaft wird dadurch ein Einführelement geschaffen mit einem elastisch expandierbaren Durchmesser, insbesondere ein mechanisch stabiler Schlauch, welches unter Krafteinwirkung seinen Durchmesser vergrößert, und bei Wegfall der einwirkenden Kraft zum Ursprungsdurchmesser zurückkehrt; ähnlich wie eine Sprungfeder.

Unter einem Umfangssegment der Hülle soll ein Bereich der Hülle verstanden werden, der als schmaler Streifen bis nahezu als komplette Mantelfläche der Hülle ausgebildet sein kann. Als Hülle soll ein Körper verstanden werden, der zusammenhängend, insbesondere materialschlüssig, aus einem oder mehreren Stoffkomponenten gebildet ist; in anderen Worten, die wenigstens zwei Arten von Umfangssegmenten einstückig miteinander ausgeführt sind. Damit kann eine besonders stabile und zuverlässige Konstruktion der Hülle erreicht werden. Hierbei soll unter "einstückig" verstanden werden, dass die Umfangssegmente der wenigstens zwei Arten nur unter Funktionsverlust zumindest eines der Umfangssegmente voneinander getrennt werden können.

Unter unterschiedlicher Elastizität soll verstanden werden, dass sich die Elastizität, insbesondere der Elastizitätsmodul, des Materials der ersten Art von Umfangssegment von dem Material der zweiten Art von Umfangssegment bei sonst gleichen Bedingungen deutlich, insbesondere mindestens um einen Faktor 2, unterscheidet. Insbesondere soll sich die Dehnbarkeit, besonders die Streckdehnung ("*elongation at yield*"), der Materialien der ersten Art von Umfangssegment und der zweiten Art von Umfangssegment bei sonst gleichen Bedingungen deutlich unterscheiden.

Eine der Arten von Umfangssegmenten ist elastisch ausgebildet. Unter elastisch soll insbesondere verstanden werden, dass das jeweilige Umfangssegment nach einer Dehnung, bei dem der Durchmesser des Einführelements vergrößert wird, reversibel wieder seine ursprüngliche Abmessungen einnimmt.

Gemäß einer günstigen Ausgestaltung kann in Umfangsrichtung die eine Art der wenigstens zwei Umfangssegmente wesentlich steifer ausgebildet sein als die andere Art der wenigstens zwei Umfangssegmente. Vorteilhaft wird erreicht, dass das Einführelement einerseits mechanisch stabil ausgebildet ist. Dadurch kann die Biegsamkeit des Einführelements im Benutzungszustand verbessert werden. Ein unerwünschtes Abknicken des Einführelements bei engen Kurvenradien, wie es bei einem Einführelement mit longitudinalem Schlitz auftreten kann, lässt sich zuverlässig vermeiden. Andererseits wird eine reversible Durchmessererweiterung ermöglicht, die ein problemloses Passieren des Implantats innerhalb des Einführelements erlaubt.

Eine günstige Ausgestaltung kann eine schlauchförmige Ausgestaltung des Einführelements vorsehen, wobei sich die wenigstens zwei Arten von Umfangssegmenten entlang einer Längserstreckung des Einführelements erstrecken können bzw. angeordnet sein können. Vorteilhaft können die jeweiligen Umfangssegmente auf der gesamten Länge des Einführelements mit, auf den Umfang bezogen, konstanten Breiten ausgebildet sein, was besonders einfach herstellbar ist. Denkbar ist jedoch auch, dass entlang der Länge des Einführelements die Breiten der verschiedenartigen und/oder gleichartigen Umfangssegmente variieren, so dass eine längenvariable Dehnbarkeit des Einführelements bereitgestellt werden kann.

Gemäß einer günstigen Ausgestaltung kann eine Art von Umfangssegment thermoplastisches Material aufweisen. Geeignete Materialien sind Polyether-ether-keton (PEEK), Polyimid (PI) Polyether-keton-keton (PEKK), Polyamide, Polyester, Polyethylen (PE), Polypropylen (PP), Polycarbonat (PC). Bevorzugt sind beispielsweise Polyamide, z. B. Polyamid 12 (PA-12, bestehend aus Laurinlactam oder ω-Aminododecansäure) oder Polyester, z. B. Polyethylenterephthalat, bekannt als PET.

Gemäß einer günstigen Ausgestaltung kann eine andere Art von Umfangssegment ein Thermoplast, der Klasse der thermoplastischen Elastomere (TPE), aufweisen. Geeignet sind thermoplastische Elastomere ausgewählt aus thermoplastischen Elastomeren auf Olefinbasis TPE-O (TPO), thermoplastischen Elastomeren auf Urethanbasis TPE-U (TPU), Styrol-Blockcopolymeren TPE-S (TPS), thermoplastischen Copolyester TPE-E oder thermoplastischen Copolyamide TPE-A (TPA) oder Kombinationen davon. Bevorzugt sind beispielsweise Polyamid-Elastomere, z. B. Polyether-Block-Amid-Block-Copolymere (PEBA), beispielsweise PEBAX (Markenname der Firma Arkema) oder Polyester-Elastomere, beispielsweise Hytrel (Markenname der Firma Dupont) oder Kombinationen davon.

Vorteilhafterweise können die wenigstens ersten und zweiten Arten von Umfangssegmenten durch miteinander kompatible Materialien gebildet sein. Diese können leicht durch einfachen Materialschluss am Schmelzpunkt zu einer geschlossenen Hülle des Einführelements zusammengefügt werden. Insbesondere kann beispielsweise ein thermoplastisches Material mit einem thermoplastischen Elastomer zusammengefügt werden, beispielsweise Polyamid PA-12 mit einem Polyamid-Elastomer oder Polyester mit einem Polyester-Elastomer.

Gemäß einer günstigen Ausgestaltung kann die Hülle mit den verschiedenartigen Umfangssegmenten durch Co-Extrusion gebildet sein. Dies ist eine besonders einfache Herstellmöglichkeit des Einführelements, bei der die unterschiedlichen, aber miteinander kompatiblen Materialien der Umfangssegmente der Hülle miteinander verbunden werden können unter gleichzeitiger Bildung der Hülle. Es ist nicht notwendig, mehrere konzentrische Materiallagen zu einer Hülle zu fügen. Ebenso wenig muss eine Nachbearbeitung erfolgen, um Öffnungen oder Schlitze in die Hülle einzuarbeiten, damit sich deren Durchmesser erweitern kann. Die entlang des Umfangs variable Elastizität der Hülle bei gleichzeitig ausreichender Festigkeit ist ein intrinsisches Merkmal der Hülle und ermöglicht ein reversibles Aufdehnen des Einführelements beim Passieren des Implantats, was ein schnelles und schonendes Einführen und Platzieren des Implantats erlaubt.

Gemäß einer günstigen Ausgestaltung kann in wenigstens einem der Umfangssegmente entlang einer Längserstreckung des Einführelements, bzw. der Hülle, wenigstens ein Stabilisierungselement angeordnet sein. Zweckmäßigerweise ist das wenigstens eine Stabilisierungselement im Bereich der Hülle angeordnet, welcher der weniger dehnbare Bereich ist und/oder im großflächigeren Bereich der Hülle (Matrix). Das Stabilisierungselement kann sich insbesondere zumindest über einen vorgegebenen Bereich durchgehend in axialer Richtung des Einführelements, bzw. der Hülle, erstrecken. Das Stabilisierungselement kann vorteilhaft beispielsweise als Draht ausgestaltet sein oder als Drahtbündel, als Faser oder Faserbündel oder eine Mischung von einem oder mehreren der Ausgestaltungen. Die Hülle kann beispielsweise am im Einbauzustand distalen Ende mit einem oder mehreren Stabilisierungselementen ausgestattet sein, und zum anderen, proximalen, Ende hin frei von Stabilisierungselementen sein. Eine umgekehrte Anordnung ist ebenfalls denkbar.

Vorteilhafterweise können die vorangehend genannten Stabilisierungselemente profiliert ausgeführt werden. Dabei wird die Profilierung bevorzugt so gestaltet, dass die innere Kontaktfläche des Stabilisierungselementes des Einführelements verringert wird. In dieser Ausgestaltung gelingt es, durch die Verkleinerung der Kontaktfläche zwischen Einführelement, insbesondere Stabilisierungselement(e), und dem einzuführenden Implantat die Reibung zu verringern, so dass sich das Implantat leichter einführen lässt. Zusätzlich kann bei gleicher Kontaktfläche durch die Profilierung des/der Stabilisierungselementes(e) die Materialmenge der einzelnen Stabilisierungselemente erhöht werden, wodurch das Einführungselement insgesamt steifer und leichter zu schieben ist.

Ebenso ist es zweckmäßig, das/die Umfangssegment(e), welche(s) die höhere Steifigkeit aufweist(en), analog zum vorangegangenen Absatz, profiliert auszugestalten.

Vorteilhaft kann die Hülle an dem (im Einbauzustand der Hülle) distalen oder proximalen Ende aus wenigstens zwei kompatiblen Materialien extrudiert werden, welche die verschiedenartigen Umfangssegmente der Hülle bilden. Das Hauptmaterial, d. h. die Matrix, wird zusätzlich mit einem Stabilisierungselement oder mit mehreren Stabilisierungselementen verstärkt, das, bzw. die, bei der Extrusion mit eingearbeitet werden, und verleiht der extrudierten Hülle vorteilhafte mechanische Eigenschaften für die Einführbarkeit der Hülle und die Verschiebeeigenschaften der Hülle entlang der Einführvorrichtung. Die Expandierbarkeit des Durchmessers der Hülle wird durch die Eigenschaften der wenigstens zwei Arten von Umfangssegmenten bewirkt.

Das medizinische Implantat, beispielweise eine selbstexpandierende Prothese wie ein Stent oder eine Herzklappe, muss an einer definierten Position platziert und von der Einführvorrichtung freigegeben werden können. Vorteilhaft kann die mit Stabilisierungselementen stabilisierte Hülle im Bedarfsfall von dem proximalen Ende der Einführvorrichtung auf den Außenschaft und in Richtung des Implantats am distalen Ende geschoben werden. Das oder die Stabilisierungselement(e) erlauben ein sicheres Aufschieben der Hülle. Bei falscher oder ungünstiger Positionierung eines teilweise am distalen Ende aus der Einführvorrichtung freigesetzten Implantats kann die Hülle über das Implantat geschoben werden und dieses überdeckt werden, und umpositioniert oder schonend wieder vollständig entfernt werden. Besonders vorteilhaft ist, dass die Oberfläche der Hülle glatt ist, so dass die Gefahr eines Verhakens vermindert ist. Scharfe Kanten, Spitzen und Schlitze sind durch die Hülle sicher abgedeckt.

Gemäß einer günstigen Ausgestaltung kann eine reibungsvermindernde Beschichtung auf der Hülle vorgesehen sein. Damit wird die Handhabung des Einführelements und seine Verwendung in einer Einführvorrichtung erleichtert und kann schneller und damit weniger belastend für einen Patienten erfolgen. Die Beschichtung kann eine hydrophobe Beschichtung sein oder eine hydrophile Beschichtung. Die Beschichtung kann auf der Innenseite der Hülle, auf der Außenseite der Hülle oder auf Innenseite und Außenseite der Hülle angeordnet sein. Sie kann auf beiden Seiten hydrophil oder auf beiden Seiten hydrophob ausgebildet sein, oder auf der Innenseite hydrophil und auf der Außenseite hydrophob, oder auf der Innenseite hydrophob und auf der Außenseite hydrophil ausgebildet sein.

Gemäß einer günstigen Ausgestaltung kann die eine Art von Umfangssegmenten als sich in Längserstreckung der Hülle erstreckender schmaler Streifen in einer als Matrix dienenden zweiten Art von Umfangssegmenten eingebettet sein. Es kann ein Streifen vorgesehen sein, oder es können zwei oder mehr Streifen vorgesehen sein. Die Zahl der Streifen kann abhängig von den verwendeten Materialien für das erste und/oder zweite Umfangssegment ausgewählt werden und/oder von erwünschten Dehnungseigenschaften der Hülle. Ebenso kann bedarfsabhängig ausgewählt werden, ob das Material mit höherer oder geringerer Elastizität in den Streifen oder in der Matrix angeordnet ist.

Gemäß einer günstigen Ausgestaltung kann die Hülle in Umfangsrichtung zumindest zwei Umfangssegmente der einen Art aufweisen, die durch jeweils ein Umfangssegment der anderen Art getrennt sind. Dies ergibt eine besonders einfache Geometrie der Hülle beim Aufweiten des Durchmessers.

Ferner wird eine Einführvorrichtung zum Einführen eines medizinischen Implantats in einen menschlichen oder tierischen Körper offenbart, umfassend zumindest ein äußeres Einführelement, das im Benutzungszustand zumindest bereichsweise elastisch aufweitbar ist, wobei das äußere Einführelement als Schleuse ausgebildet ist, durch welche ein Außenschaft, der einen Innenschaft umgibt, auf welchem Innenschaft ein medizinisches Implantat zum Einführen in einen menschlichen oder tierischen Körper angeordnet ist, an einen Einsatzort einführbar ist. Das äußere Einführelement umfasst dabei eine Hülle, welche in Umfangsrichtung wenigstens zwei Arten von Umfangssegmenten mit unterschiedlicher Elastizität aufweist. Durch die Dehnbarkeit des Einführelements bei Durchführung des Implantats bzw. eines Katheters mit Implantat kann die Öffnung im Körper zur Einführung des Implantats kleiner ausfallen, als wenn eine Schleuse mit maximal benötigtem Durchmesser eingesetzt würde.

Gemäß einem weiteren Aspekt der Erfindung wird eine Einführvorrichtung zum Einführen eines medizinischen Implantats in einen menschlichen oder tierischen Körper vorgeschlagen, umfassend zumindest ein äußeres Einführelement, wobei das äußere Einführelement zumindest als distales Ende eines Außenschafts ausgebildet ist, der einen Innenschaft umgibt, und der ein auf dem Innenschaft angeordnetes medizinisches Implantat zumindest vor dem oder beim Einführen in einen menschlichen oder tierischen Körper abdeckt, wobei das äußere Einführelement im Benutzungszustand zumindest bereichsweise elastisch aufweitbar ist. Das äußere Einführelement umfasst dabei eine Hülle, welche in Umfangsrichtung wenigstens zwei Arten von Umfangssegmenten mit unterschiedlicher Elastizität aufweist. Durch seine Dehnbarkeit kann das Einführelement, d. h. der Außenschaft, über ein fehlpositioniertes Implantat, beispielsweise einen selbstexpandierenden Stent, geschoben werden, so dass dieser repositioniert oder gefahrlos wieder aus dem Körper entfernt werden kann. Weist das Einführelement einen Bereich auf, in dem wenigstens bereichsweise in wenigstens einem der Umfangssegmente entlang einer Längserstreckung der Hülle wenigstens ein Stabilisierungselement angeordnet ist, kann das Zurückschieben des Außenschafts besonders einfach und stabil erfolgen. Selbst wenn eine Repositionierung des Implantats fehlschlagen würde, umgibt das wieder aufgeschobene Einführelement das Implantat, so dass dieser weniger Mikroverletzungen in den Gefäßen verursacht, wenn das Implantat wieder entfernt wird.

Ferner wird offenbart, dass optional die Einführvorrichtungen nach den verschiedenen Aspekten der Erfindung auch kombiniert werden können, so dass sich eine vorteilhafte Einführvorrichtung ergibt, deren Außenschaft ein distales Ende aufweist, das zumindest bereichsweise elastisch aufweitbar ist, und welche Einführvorrichtung durch eine elastisch aufweitbare Schleuse eingeführt werden kann. Dabei haben die Schleuse und zumindest das distale Ende des Außenschafts wenigstens eines der Merkmale des vorstehend beschriebenen Einführelements.

Günstigerweise kann bei allen vorgeschlagenen Einführvorrichtungen das äußere Einführelement einen Bereich aufweisen, in dem wenigstens bereichsweise, in wenigstens einem der Umfangssegmente entlang einer Längserstreckung der Hülle, wenigstens ein Stabilisierungselement angeordnet ist. Dadurch ist das Einführelement besonders stabil.

Die Erfindung ist nachfolgend beispielhaft, anhand von in Zeichnungen dargestellten Ausführungsbeispielen, näher erläutert. Es zeigen in schematischer Darstellung:
- Fig. 1A, 1B: eine Seitenansicht (Fig. 1A) und einen Querschnitt (Fig. 1B) eines ersten Ausführungsbeispiels eines Einführelements nach der Erfindung; und
- Fig. 2A, 2B: eine Seitenansicht (Fig. 2A) und einen Querschnitt (Fig. 2B) eines weiteren Ausführungsbeispiels eines Einführelements nach der Erfindung;
- Fig. 3A, 3B: eine Seitenansicht (Fig. 3A) und einen Querschnitt (Fig. 3B) eines weiteren Ausführungsbeispiels eines Einführelements nach der Erfindung;
- Fig. 4: eine Seitenansicht einer beispielhaften Ausgestaltung einer Einführvorrichtung nach der Erfindung;
- Fig. 5A, 5B: in einer Seitenansicht eine Einführsituation einer Einführvorrichtung nach einer Ausgestaltung der Erfindung mit teilweise freigesetztem Implantat (Fig. 5A) und mit wieder abgedecktem Implantat mit aufgeschobenem Einführelement (Fig. 5B); und
- Fig. 6A, 6B: eine Seitenansicht (Fig. 6A) und einen Querschnitt (Fig. 6B) eines weiteren Ausführungsbeispiels des Einführelementes nach der Erfindung mit profilierten Stabilisierungselementen.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

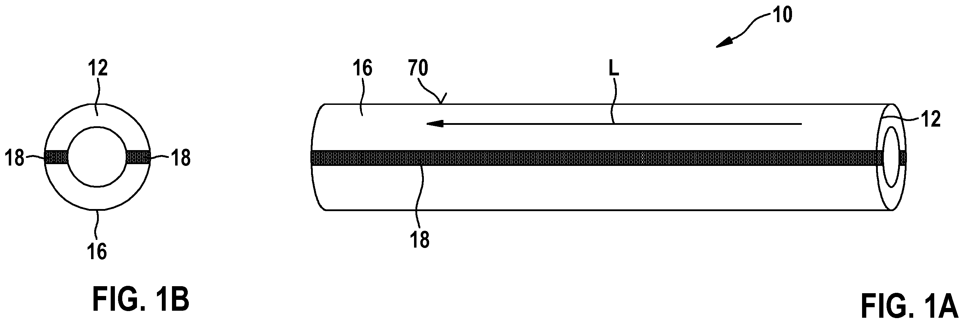

Die Figuren 1A, 1B zeigen eine erste beispielhafte Ausgestaltung eines Einführelements 10 als Seitenansicht (FIG. 1A) und als Querschnitt (FIG. 1B). Das Einführelement 10 kann insbesondere beispielsweise als Schleuse 80 für einen Katheter eingesetzt werden, oder auch unmittelbarer Bestandteil eines Katheters sein, etwa eines Außenschafts eines Katheters.

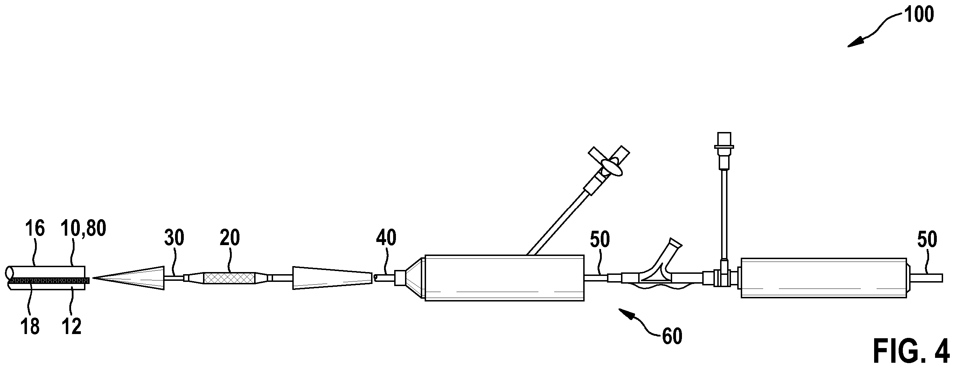

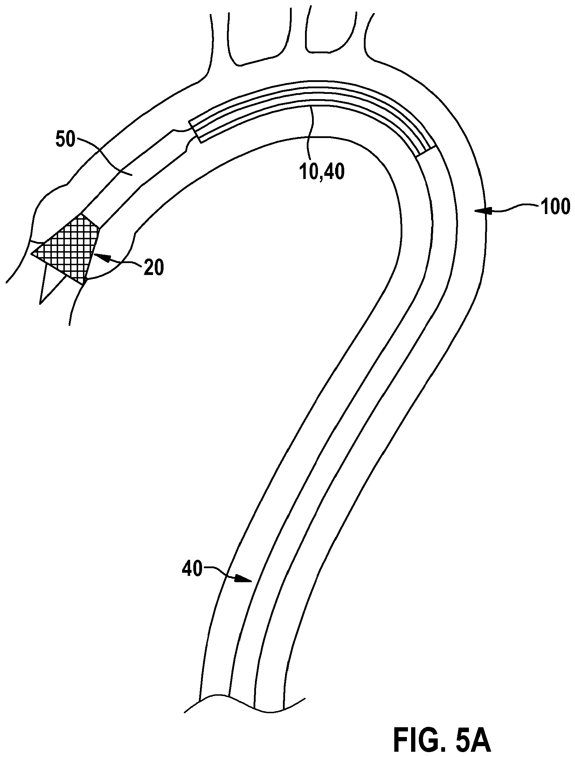

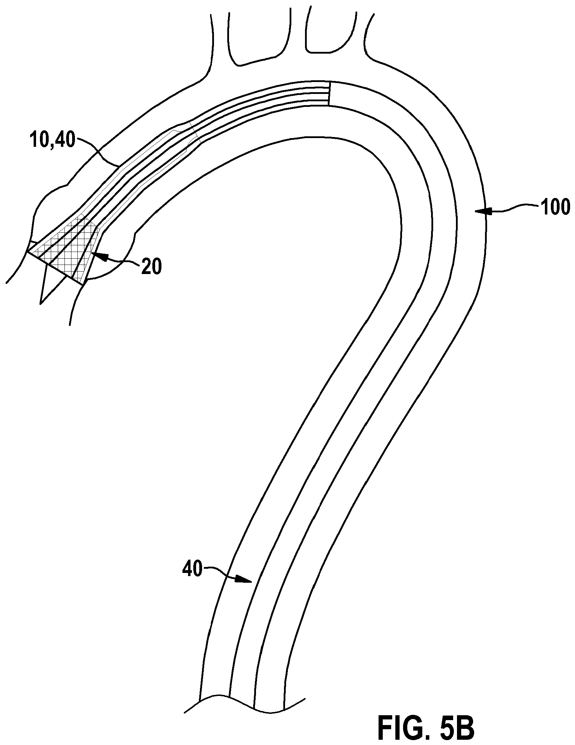

Das Einführelement 10 ist zum Zusammenwirken mit einer Einführvorrichtung 100 (Figuren 4, 5A, 5B) zum Einführen eines medizinischen Implantats 20, z. B. einen selbstexpandierenden Stent, in einen menschlichen oder tierischen Körper ausgebildet. Die schlauchförmige Hülle 12 des Einführelements 10 weist in Umfangsrichtung wenigstens zwei Umfangssegmente 16, 18 einer ersten und einer zweiten Art mit unterschiedlicher Elastizität auf, die sich z. B. diametral gegenüberliegen. Das zweite Umfangssegment 18 ist als schmaler Streifen entlang der Längserstreckung L des Einführelements 19 ausgebildet.

Das erste Umfangssegment 16 ist wesentlich steifer das zweite Umfangssegment 18. Das erste, steife Umfangselement 16 ist beispielsweise aus Polyamid PA 12 gebildet, und das zweite, elastische Umfangssegment 18 aus Polyamid-Elastomer.

Das Einführelement 10 kann optional eine reibungsvermindernde Beschichtung 70 aufweisen, etwa auf der Außenseite der Hülle 12. Die Beschichtung 70 kann hydrophile oder hydrophobe Eigenschaften aufweisen.

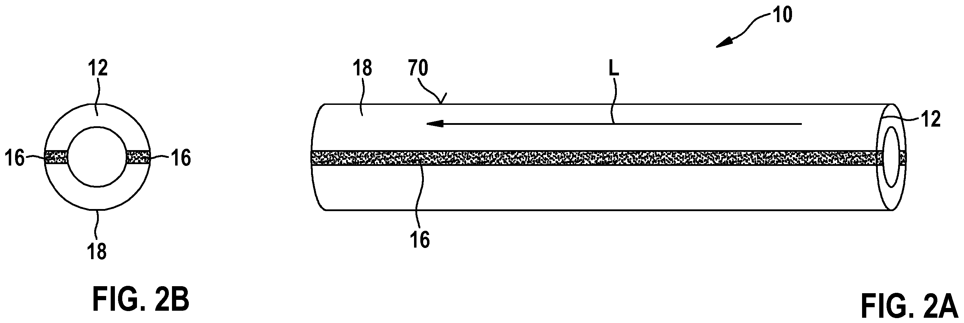

Die Figuren 2A, 2B zeigen eine weitere beispielhafte Ausgestaltung eines Einführelements 10 als Seitenansicht (Figur 2A) und als Querschnitt (Figur 2B).

Das Einführelement 10 ist zum Zusammenwirken mit einer Einführvorrichtung 100 (Figuren 4, 5A, 5B) zum Einführen eines medizinischen Implantats 20 in einen menschlichen oder tierischen Körper ausgebildet. Die schlauchförmige Hülle 12 des Einführelements 10 weist in Umfangsrichtung wenigstens zwei Umfangssegmente 16, 18 mit unterschiedlicher Elastizität auf, die sich z. B. jeweils diametral gegenüberliegen. Das zweite Umfangssegment 18 ist als breiter Streifen entlang der Längserstreckung L des Einführelements 10 ausgebildet, in den das erste Umfangssegment 16 als schmaler Streifen eingebettet ist.

Das erste Umfangssegment 16 ist wesentlich steifer das zweite Umfangssegment 18. Das erste Umfangselement 16 ist beispielsweise aus Polyester gebildet, und das zweite Umfangssegment 18 aus Polyester-Elastomer.

Das Einführelement 10 kann optional eine reibungsvermindernde Beschichtung 70 aufweisen, etwa auf der Außenseite der Hülle 12. Die Beschichtung 70 kann hydrophile oder hydrophobe Eigenschaften aufweisen.

Die in den Ausführungsbeispielen gezeigten Umfangssegmente 16, 18 der Hülle 12 sind vorteilhaft durch miteinander kompatible Materialien gebildet, aus denen sich die Hülle 12 besonders leicht durch Co-Extrusion herstellen lässt. Die streifenförmigen Umfangssegmente 16 oder 18 sind in die halbschalenförmigen Umfangssegmente 18 oder 16 wie in eine Matrix eingebettet. Die Umfangssegmente 16, 18 bilden zusammen einen in Umfangsrichtung geschlossenen Hüllenmantel, der reversibel aufgeweitet werden kann, wenn das Implantat 20 (Figur 1) die betreffende Stelle innerhalb des Einführelements 10 passiert.

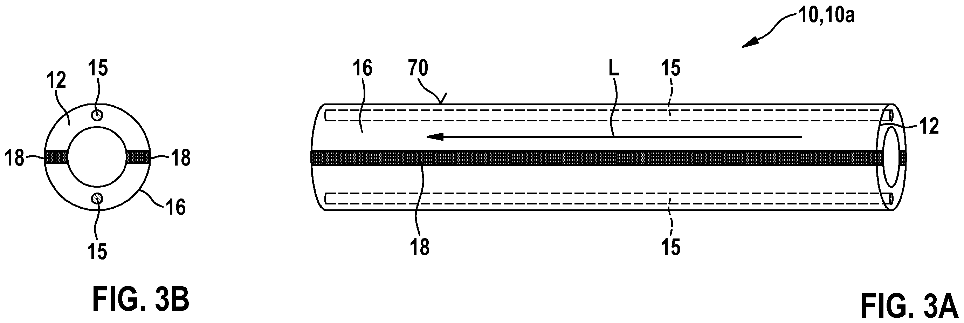

Die Figuren 3A, 3B zeigen eine weitere beispielhafte Ausgestaltung eines Einführelements 10 als Seitenansicht (Figur 3A) und als Querschnitt (Figur 3B). Das Einführelement 10 weist wenigstens bereichsweise (Bereich 10a) in wenigstens einem der Umfangssegmente 16, vorzugsweise im Bereich mit geringerer Dehnbarkeit, entlang einer Längserstreckung L der Hülle 12 wenigstens ein Stabilisierungselement 15 auf, etwa ein Draht, insbesondere ein metallischer Draht. Das Stabilisierungselement 15 unterstützt die mechanische Stabilität des Einführelements 10. Stabilisierungselemente 15 können grundsätzlich in einem oder mehreren oder allen Umfangssegmenten 16, 18 vorgesehen sein, auch in verschiedenartigen Umfangssegmenten 16, 18 mit unterschiedlicher Elastizität.

Günstige Materialien für die steifen (wenig elastischen) Umfangssegmente 16 nach den Figuren 1, 2, 3 sind PEEK, PI, PE, PP, PC, bevorzugt Polyamide wie etwa PA12 (z.B. Grilamid L25) oder Polyester etwa PET sowie Polyamid-Elastomere mit Härten von Shore D > 60 oder Polyester-Elastomere mit Härten von Shore D > 60. Materialien für die elastischen Umfangssegmente 18 nach den Figuren 1, 2, 3 sind TPE-O, TPE-U, bevorzugt Polyamid-Elastomere (TPE-A) mit Härten von Shore D < 70 oder Polyester-Elastomere (TPE-E) mit Härten von Shore D < 70. Dabei sind die elastischen Unterschiede zwischen den Umfangssegmenten 16 und 18 bevorzugt deutlich ausgeprägt.

Figur 4 zeigt zur Veranschaulichung der Erfindung eine Seitenansicht eines ersten Ausführungsbeispiels einer Einführvorrichtung 100, die hier z.B. einen Katheter umfasst, mit der ein selbstexpandierender Stent eingeführt werden soll. Die Einführvorrichtung 100 umfasst innere und äußere Einführelemente in Form eines Innenschafts 50 und eines diesen umgebenden Außenschafts 40, an dessen proximalen Enden eine übliche, nicht näher beschriebene Handhabeeinrichtung 60 mit verschiedenen Anschlusstücken, z.B. zum Spülen der Lumen von oder zwischen Innenschaft 50 und Außenschaft 40 und zum Freigeben des Implantats 20, angeordnet ist. Zwischen den entgegengesetzt gelegenen distalen Enden ist ein Implantat 20 mit einer üblichen Führungseinrichtung 30 auf dem Innenschaft 50 angeordnet.

Zum Einführen des Katheters bzw. des Ensembles von Außenschaft 40, dem vom Außenschaft 40 umgebenen Innenschaft 50, das auf dem Innenschaft 50 angeordnete Implantat 20 mit Führungseinrichtung 30, in den menschlichen oder tierischen Körper wird dieser mit dem Implantat 20 bzw. der Führungseinrichtung 30 voran durch ein als Schleuse 80 ausgebildetes Einführelement 10 geschoben. Zur Einführung des Katheters wird zunächst eine flexible Schleuse 80, z. B. in das Blutgefäß, eingeführt, wodurch der Katheter in das Blutgefäß gleiten kann. Das als Schleuse 80 ausgebildete Einführelement 10 umfasst wie in den vorstehend aufgeführten Ausführungsbeispielen beschrieben eine Hülle 12 mit einem Umfangssegment 16 einer ersten Art und einem Umfangssegment 18 einer zweiten Art, wobei sich die beiden Arten in ihrer Elastizität unterscheiden. Das Umfangssegment 18 der zweiten Art ist entlang der Längserstreckung des schlauchförmig ausgebildeten Einführelements 10 als schmaler Streifen ausgebildet, der im matrixartigen Umfangssegment 16 der ersten Art eingebettet ist.

Die Figuren 5A und 5B zeigen zur Veranschaulichung der Erfindung eine Seitenansicht eines weiteren Ausführungsbeispiels einer Einführvorrichtung 100, bei der ein Einführelement 10 als distales Ende eines Außenschafts 40 vorgesehen ist. Das Einführelement 10 umfasst wie in den vorstehend aufgeführten Ausführungsbeispielen beschrieben eine Hülle 12 mit einem Umfangssegment 16 einer ersten Art und einem Umfangssegment 18 einer zweiten Art, wobei sich die beiden Arten in ihrer Elastizität unterscheiden. Das Umfangssegment 18 der zweiten Art ist entlang der Längserstreckung des schlauchförmig ausgebildeten Einführelements 10 als schmaler Streifen ausgebildet, der im matrixartigen Umfangssegment 16 der ersten Art eingebettet ist. Der Außenschaft 40 kann vollständig als Einführelement 10 ausgebildet sein, oder nur bereichsweise, insbesondere am distalen Ende des Außenschafts 40 in dem Bereich, der das Implantat 20 überdeckt.

Besonders vorteilhaft kann hier ein wie in Fig. 3A, 3B beschriebenes Stabilisierungselement 15 in der Hülle 12 vorgesehen sein. An den dergestalt stabilisierten Bereich der Hülle 12 kann sich beispielsweise ein nicht zusätzlich stabilisierter Bereich der Hülle 12 anschließen. In Fig. 5A ist in einer Seitenansicht eine Einführsituation der Einführvorrichtung 100 mit teilweise freigesetztem Implantat 20 in Form eines selbstexpandierenden Stents am distalen Ende der Einführvorrichtung 100 dargestellt.

In Fig. 5B ist in einer Seitenansicht die Einführsituation einer Einführvorrichtung 100 mit wieder abgedecktem Implantat 20 mit aufgeschobenem Einführelement 10 dargestellt. Das Einführelement 10 kann sich über die ganze axiale Länge der Einführvorrichtung 100 bzw. des Außenschafts 40 erstrecken, wobei vorteilhaft an dessen distalen Ende ein Bereich 10a mit einem oder mehreren Stabilisierungselementen 15 vorgesehen sein kann.

Um das teilweise freigesetzte Implantat 20 beispielsweise zu repositionieren, kann das Einführelement 10 vom proximalen Ende der Einführvorrichtung 100 in distaler Richtung aufgeschoben werden, bis der stabilisierte Bereich 10a des Einführelements 10 am distalen Ende des Außenschafts 40 über das teilweise freigesetzte Implantat 20 geschoben ist und dieses wieder vollständig abdeckt. Gegebenenfalls kann das Implantat 20 in diesem Zustand auch wieder schonend aus dem Körper entfernt werden, da das Einführelement 10 scharfe Kanten oder spitze Teile des Implantats abdeckt.

Ferner wird offenbart, dass das erfindungsgemäße Einführelement 10 vorteilhaft als Schleuse 80 eingesetzt werden kann, um die Einführvorrichtung 100 in einen menschlichen oder tierischen Körper einzuführen, sowie zusätzlich oder alternativ als Außenschaft 40 oder Teil des Außenschafts 40 zum Wiederabdecken ("*Re-Sheathing*") eines teilweise freigesetzten Implantats 20 eingesetzt werden.

Vorteilhaft können durch die Verwendung geeigneter Materialien für die Umfangssegmente 16, 18 die dehnbareren der Umfangssegmente 16, 18 beispielsweise eine bis zu viermal höhere Dehnung aufweisen als die weniger dehnbaren Umfangssegmente 16, 18.

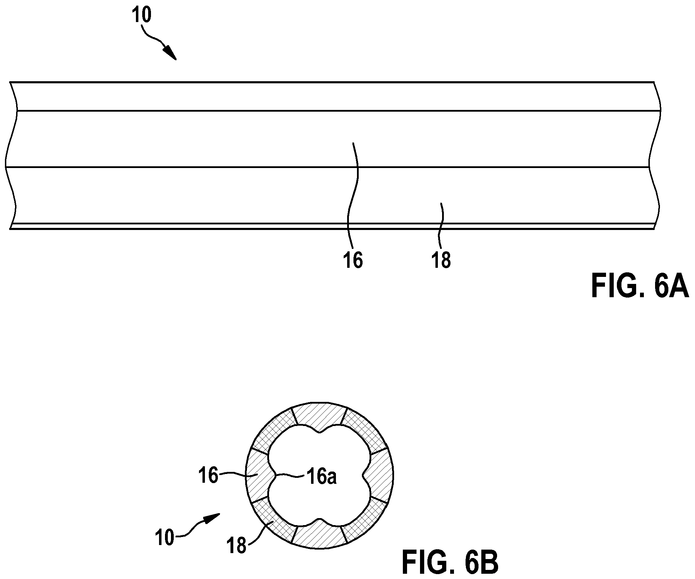

Fig. 6A ist eine Seitenansicht eines Ausführungsbeispiels eines Einführungselementes 10, wie es beispielsweise in Zusammenwirkung mit einer Einführvorrichtung 100 zur Einführung eines Implantates 20 in einen menschlichen oder tierischen Körper verwendet werden kann.

In Fig. 6B wird das Ausführungsbeispiel eines Einführelementes nach Fig. 6A im Querschnitt dargestellt.

Es wird offenbart, dass das Einführelement 10 so beispielsweise als Schleuse 80 für einen Katheter verwendet werden kann. Das Einführungselement 10 dieses Ausführungsbeispiels weist wenigstens zwei verschiedene Umfangselemente 16, 18 einer ersten und einer zweiten Art mit unterschiedlicher Elastizität auf. In diesem Ausführungsbeispiel weisen die Umfangselemente 16 eine höhere Steifigkeit und eine geringere Elastizität als die Umfangselemente 18 auf. Bei diesem Ausführungsbeispiel sind die Umfangselemente 16 als profilierte Metallstreifen ausgebildet und in ein Polyamid-Elastomer eingebettet, welches die Umfangselemente 18 bildet.

Wie in Fig. 6B dargestellt, sind die Umfangselemente 16 derart profiliert, dass sich eine Spitze 16a im Inneren des Einführelementes 10 ausgebildet hat. Dadurch wird die Kontaktfläche zwischen dem beispielsweise als Schleuse 80 funktionierenden Einführelementes 10 und dem einzuführenden Katheter verringert. Dadurch lässt sich ein leichteres Gleiten des Katheters im Einführelement 10 erreichen. Gleichzeitig kann durch die Vergrößerung der Materialmenge des Umfangselementes 16 gegenüber einer nicht profilierten Ausgestaltung die Steifigkeit des gesamten Einführelementes 10 erhöht werden, wodurch das Einführelement selbst leichter geschoben werden kann.

Das in den Figuren 6A und 6B gezeigte Einführelement 10 kann auch in einer Einführvorrichtung 100 als distales Ende des Außenschafts 40 analog zu den Figuren 5A und 5B verwendet werden.

## Patentansprüche

1. Einführvorrichtung (100) zum Einführen eines medizinischen Implantats (20) in einen menschlichen oder tierischen Körper, wobei das medizinische Implantat (20) als eine selbstexpandierende Prothese ausgebildet ist, umfassend zumindest ein äußeres Einführelement (10), wobei das äußere Einführelement (10) zumindest als distales Ende eines Außenschafts (40) ausgebildet ist, der einen Innenschaft (50) umgibt, und der ein auf dem Innenschaft (50) angeordnetes medizinisches Implantat (20) zumindest vor dem oder beim Einführen in einen menschlichen oder tierischen Körper abdeckt, wobei das äußere Einführelement (10) im Benutzungszustand zumindest bereichsweise elastisch aufweitbar ist, **dadurch gekennzeichnet, dass** das Einführelement eine Hülle umfasst, welche in Umfangsrichtung wenigstens zwei Arten von Umfangssegmenten (16, 18) mit unterschiedlicher Elastizität aufweist, wobei ein Umfangssegment der Hülle ein Bereich der Hülle ist, der als schmaler Streifen bis nahezu als komplette Mantelfläche der Hülle ausgebildet ist, und wobei das zweite Umfangssegment (18) als breiter Streifen entlang der Längserstreckung L des Einführelements (10) ausgebildet ist, in den das erste Umfangssegment (16) als schmaler Streifen eingebettet ist, und wobei das erste Umfangssegment (16) wesentlich steifer ist als das zweite Umfangssegment (18) und profiliert ausgestaltet ist.

2. Einführvorrichtung (100) nach Anspruch 1, **dadurch gekennzeichnet, dass** die selbstexpandierende Prothese ein Stent oder eine Herzklappe ist.

3. Einführvorrichtung (100) nach Anspruch 1, **dadurch gekennzeichnet, dass** die wenigstens zwei Arten von Umfangssegmenten (16, 18) sich entlang einer Längserstreckung (L) des Einführelements (10) erstrecken.

4. Einführvorrichtung (100) nach einem der vorhergehenden Ansprüche, **dadurch gekennzeichnet, dass** das Einführelement eine reibungsvermindernde Beschichtung (70) aufweist.

5. Einführvorrichtung nach einem der vorhergehenden Ansprüche, **dadurch gekennzeichnet, dass** das äußere Einführelement (10) einen Bereich (10a) aufweist, in dem wenigstens bereichsweise in wenigstens einem der Umfangssegmente (16, 18) entlang einer Längserstreckung der Hülle (12) wenigstens ein Stabilisierungselement (15) angeordnet ist.

## Claims

1. A delivery device (100) for inserting the medical implant (20) into a human body or an animal body, the medical implant (20) being designed as a self-expanding prosthesis, comprising at least one outer delivery element (10), the outer delivery element (10) being at least designed as a distal end of an outer shaft (40) surrounding an inner shaft (50) and covering a medical implant (20), which is arranged on the inner shaft (50), at least prior to or during insertion into a human body or an animal body, the outer delivery element (10), in the usage state, being at least regionally elastically dilatable, **characterized in that** the delivery element comprises a sleeve that, in the circumferential direction, comprises at least two types of circumferential segments (16, 18) having differing elasticities, one circumferential segment of the sleeve being a region of the sleeve that is designed as a narrow strip to almost as the entire lateral surface of the sleeve, and the second circumferential segment (18) being designed as a wide strip along the longitudinal extension L of the delivery element (10) into which the first circumferential segment (16) is embedded as a narrow strip, and the first circumferential segment (16) being designed to be considerably more rigid than the second circumferential segment (18) and to be profiled.

2. The delivery device (100) according to claim 1, **characterized in that** the self-expanding prosthesis is a stent or a heart valve.

3. The delivery device (100) according to claim 1, **characterized in that** the at least two types of circumferential segments (16, 18) extend along a longitudinal extension (L) of the delivery element (10).

4. The delivery device (100) according to any one of the preceding claims, **characterized in that** the delivery element comprises a friction-lowering coating (70).

5. The delivery device according to any one of the preceding claims, **characterized in that** the outer delivery element (10) includes a region (10a) in which at least one stabilizing element (15) is arranged, at least regionally, in at least one of the circumferential segments (16, 18) along a longitudinal extension of the sleeve (12).

## Revendications

1. Dispositif d'insertion (100) permettant d'insérer un implant médical (20) dans un corps humain ou d'un animal, l'implant médical (20) étant conçu sous forme de prothèse auto-expansible comprenant au moins un élément d'insertion extérieur (10), où l'élément d'insertion extérieur (10) est conçu sous forme d'une extrémité distale d'une tige extérieure (40) qui entoure une tige intérieure (50), et qui recouvre l'implant médical (20) disposé sur la tige intérieure (50) au moins avant ou pendant l'insertion dans un corps humain ou d'un animal, où l'élément d'insertion extérieur (10) peut être élargi de manière élastique au moins par endroits à l'état opérationnel, **caractérisé en ce que**
l'élément d'insertion comprend une enveloppe, laquelle présente au moins deux types de segments circonférentiels (16, 18) d'élasticités différentes dans la direction circonférentielle, où un segment circonférentiel de l'enveloppe est une zone de l'enveloppe qui est conçue sous forme d'une bande mince jusqu'à pratiquement la surface d'enveloppe complète de l'enveloppe, et où le deuxième segment circonférentiel (18) est conçu sous forme d'une bande large le long de l'axe d'extension longitudinale L de l'élément d'insertion (10) dans lequel le premier segment circonférentiel (16) est incorporé sous forme de bande fine et où le premier segment circonférentiel (16) est essentiellement plus rigide que le deuxième segment circonférentiel (18) est conçu profilé.

2. Dispositif d'insertion (100) selon la revendication 1, **caractérisé en ce que** la prothèse auto-expansible est un stent ou une valve cardiaque.

3. Dispositif d'insertion (100) selon la revendication 1, **caractérisé en ce que** les au moins deux types de segments périphériques (16, 18) s'étendent le long d'une extension longitudinale (L) de l'élément d'insertion (10).

4. Dispositif d'insertion (100) selon l'une des revendications précédentes, **caractérisé en ce que** l'élément d'insertion présente un revêtement (70) diminuant la friction.

5. Dispositif d'insertion (100) selon l'une des revendications précédentes, **caractérisé en ce que** l'élément d'insertion extérieur (10) présente une zone (10a) dans laquelle au moins un élément de stabilisation (15) est disposé au moins par endroits dans l'au moins un des segments circonférentiels (16, 18) le long d'une extension longitudinale de l'enveloppe (12).
